# EUROPEAN PATENT SPECIFICATION

(11) **EP 2 520 248 B1**
(45) Date of publication and mention of the grant of the patent: **26.09.2018**
(21) Application number: 10840615.8
(22) Date of filing: 17.12.2010
(51) Int. Cl.: A61F 2/18

(54) **PROSTHESIS FOR COMPLETE OSSICULAR REPLACEMENT**
PROTHESE FÜR KOMPLETTEN TROMMELFELLERSATZ
PROTHÈSE DE SUBSTITUTION OSSICULAIRE TOTALE

(30) Priority: 30.12.2009 ES 200902423 P
(43) Date of publication of application: 07.11.2012
(73) Proprietor: Universidad De Valladolid (40%), 47002 Valladolid (ES)
(72) Inventor: VALLEJO VALDEZATE, Luis Ángel, 47002 Valladolid (ES)
(74) Representative: Rodriguez Perez, Jesus
(86) International application number: PCT/ES2010/000522
(87) International publication number: WO 2011/080363

(56) References cited:
- EP-A2- 1 498 088
- ES-T3- 2 303 005
- US-A- 4 655 776
- US-A- 5 061 280
- US-A- 5 514 177
- US-A1- 2002 095 063
- US-A1- 2007 021 833
- US-A1- 2008 097 602
- US-A1- 2008 208 337
- US-B1- 6 203 571
- US-B2- 7 011 683

## Description

### Object of the invention

The present invention is defined in claim 1 and it relates to a total ossicular replacement prosthesis, envisaged to replace damaged middle-ear ossicles in order to enable the recovery of hearing which would otherwise be partially lost.

Therefore, the object of the invention is to provide a prosthesis which enables partial repair of the ossicular damage, mitigating hypoacusia or hearing loss to a certain degree.

The prosthesis falls within the medicinal sector, specifically under otolaryngology (field of otology).

### Background of the invention

There are several pathological processes (infections, tumours, etc.) that affect the human middle ear and which, in themselves or due to the surgical treatment they require, cause partial or total damage to the ossicular chain, altering the normal mechanism of transmission of sound in the ear and causing varying degrees of hearing loss, commonly known as hypoacusia, in accordance with the damage caused, which depends on the number of affected ossicles.

In order to mitigate this situation and replace the damaged ossicles of the middle ear, many authors have designed small prostheses which, implanted between the healthy ossicles or between the eardrum and the ossicles, enable the partial repair of ossicular damage, and mitigate the hypoacusia to a certain degree

Further, having observed and analysed the mechanical-acoustic behavior of different ossicular replacement prostheses in the state of art, it has been proven that most, due to their design or constituent material, do not have the desired acoustic efficiency. Some of the most important deficiencies include the following:
- Deficiencies arising from the construction material. Ossicular replacement prostheses are manufactured from different materials, all of which are biocompatible, such as platinum, titanium, ceramic materials and hydroapatite (similar to bone). Prostheses manufactured from ceramic materials or materials similar to bone tend to move from their initial position, coming into contact with the adjacent bone, therefore suppressing their acoustic efficiency. In this regard, the most useful prostheses are those manufactured from metals such as platinum and particularly titanium, due to being elements with high rigidity and low mass.
- Design deficiencies. To date, prosthesis design has not been based on mechanical-acoustic efficiency criteria, but rather on the preferences of the surgeons who design them (their surgical dexterity, conception of middle-ear mechanics, etc.). This has led to the proliferation of designs with very disparate mechanical behaviours. Also, given the impossibility of fixing the prosthesis to the middle ear, it remains "loose" inside the eardrum until it is stabilised by the normal healing process. But this does not always happen, due to which ossicular replacement prostheses tend to move from their initial position.
- Finally, the prostheses of the state of the art have not been designed taking into account current ear physiology concepts, such as the important function of the middle-ear muscles. Therefore, currently known prostheses do not leverage the functions of the ear muscles, resulting in less voice discrimination in noisy environments.

However, some prostheses, such as those disclosed in DE 203 10609 and ES 2 285 328, envisaged for being anchored to the hammer handle by means of a clip-type anchor are known to exist, although the design of the prostheses corresponding to said patents is deficient as the tympanic membrane of the hammer handle must be lifted for implantation, which affects vascularisation thereof, whereupon said hammer handle eventually breaks.

The US2008097602 discloses an ossicular prosthesis, comprising: a flat head portion for mating with the tympanic membrane; a superelastic metal alloy engagement structure for providing a compressive force about a portion of an ossicle; and a shaft extending between said head portion and said engagement structure and having a length not exceeding approximately 5 mm, wherein said engagement structure defines an opening and is deformable to widen the opening to permit the portion of the ossicle to be received therein, and when the engagement structure is deformed to receive the portion of the ossicle the stress in the engagement structure, after a short duration of linear elastic behavior, remains substantially constant, according to the preamble of claim 1.

### Description of the invention

The total ossicular replacement prosthesis of the invention solves the previously expounded problem, as it is specially designed for anchoring underneath and at the middle part of the hammer handle, which are essential features of the invention, and extending towards the oval window, in such a manner that the distal end of the prosthesis is introduced directly in the vestibule (inner ear) or, preferably, rests upon a perichondrial or vein graft, avoiding the creation of perilymphatic fistula.

More specifically, the prosthesis of the invention has an elongated and slightly arched body or member, one of the ends of which finishes in a type of U-shaped anchoring fork, having elastic capacity which, in such a manner that the arms are separated at their ends in order to adapt and adjust perfectly to the hammer handle.

With regard to the arching of the member, it is envisaged to extend from the crossing point of the fork arms or U-shaped end of the anchor to the distal end of the member, said distal end consisting of a portion by way of extension of the member, the thickness of which increases progressively.

With regard to the material, although it can be manufacturated from biocompatible plastics as teflon or fluoroplastic, it shall preferably be manufactured from a metal such as nitinol or titanium, particularly the latter, as titanium has proven to be the component best accepted in the middle ear, with negligible extruding rates, due to which it is an ideal material although, as mentioned earlier, the prosthesis can be manufactured from other materials.

That is, titanium is a biocompatible material, the rigidity and low mass of which makes it ideal for manufacturing the ossicular replacement prosthesis object of the invention.

With regard to the design, it allows anchorage to the hammer handle, which is essential as it prevents movement thereof as a result of the healing or fibrotic phenomena following all surgical processes, in such a manner that said movement of the prosthesis subsequent to implantation thereof enables it to come into contact with the eardrum, functionally disabling it, due to which, on having a special design and preventing the movement thereof when implanted, the problems of conventional prosthesis that are usually disposed either underneath the upper rear quadrant of the tympanic membrane or underneath the hammer handle, but not anchored to any ossicular element.

Furthermore, the prosthesis design leverages the action of one of the middle-ear muscles, specifically the hammer muscle, thereby improving the discrimitation of sound in noisy environments, which is considered a mechanical-acoustic advantage.

Ultimately, the prosthesis of the invention leverages the biological and mechanical-acoustic advantages of anchorage to the hammer handle, as well as preserving the vascularisation of said ossicular element due to be anchored underneath it, and not having to lift the tympanic membrane, thereby preventing the eventual breakage thereof.

Additionally, on anchoring the prosthesis to the hammer handle rather than leaving it "loose" as occurs traditionally in the case of the middle ear, the biological characteristics of the contraction of the tympanic tensor muscle are leveraged, making the prosthesis in question especially suited for improving the intelligibility of sound in noisy environments.

### Description of the drawings

In order to complement the following description and to help better understand the characteristics of the invention, in accordance with a preferred embodiment thereof, a set of drawings is attached wherein the following has been represented in an illustrative and non-limiting manner:
Figure 1 shows a general view of a total ossicular replacement prosthesis, in accordance with the object of the invention.
Figure 2 shows a perspective view of the anchorage of the prosthesis of the preceding figure, extending from the hammer handle to the corresponding oval window.

### Preferred embodiment of the invention

As can be observed in the aforementioned figures, the total ossicular replacement prosthesis object of the invention, preferably manufactured from titanium, without ruling out other biocompatible materials, has an arched member (1), the proximal end of which finishes in a fork or U-shaped configuration (2), while the distal end thereof finishes in a section (3) by way of extension of the member (1). Said section (3) is called the base and increases progressively in thickness towards its free end, constituting an extension of the member (1), which is arched and has the function expounded below.

The length of the arched member (1) corresponds to reference (4), while the length of the base (3) corresponds to reference (5) and the length of the fork (2) corresponds to reference (6).

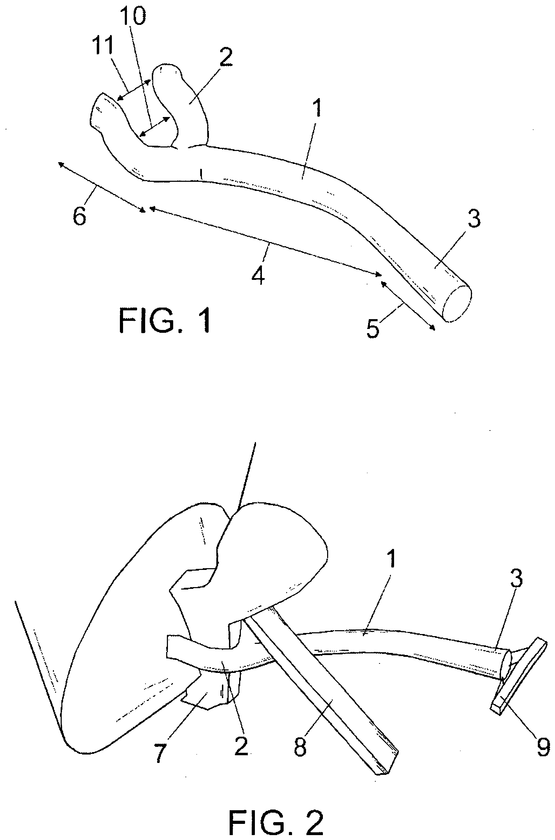

The fork (2) corresponding to the proximal end is elastic and is designed to be anchored to medial part of eardrum handle (7), underneath it, without lifting the tympanic membrane, as shown in figure 2, being anchored and fitted into place to prevent subsequent movement thereof.

The arched shape of the member (1) has been envisaged to overcome the eccentric position of the hammer muscle (8) with respect to the centre of the vestibule.

The section that defines the base (3), the widening thereof, has been envisaged to increase the surface area in contact with the vestibule perilymph (9).

As can be observed, the fork (2) has a section (10) of greater width than section (11) for the purpose of ensuring effective anchorage, to which end the arms corresponding to this fork are slightly divergent so as to achieve prefect adaptation in the anchorage thereof. This difference in amplitude in the width of the fork (2), corresponding to sections (10 and 11) allows clip-type anchorage, which is achieved by simply applying pressure.

Accordingly and as a conclusion of the above essential features of the invention, the prosthesis for complete ossicular replacement which, being envisaged to replace all the damaged ossicles of the middle ear, for the purpose of mitigating hypoacusia or hearing loss to a certain extent, having a member (1) which extends at one of its ends, considered proximal, into a fork (2) for anchorage thereof and, at the opposite end, into a base (3) having progressively increasing width, has the following essential characteristics: the member (1) is a continuously arched member (1) for anchorage thereof to the corresponding medial zone of the hammer handle (7) underneath it without lifting the tympanic membrane, while the base (3) establishes a larger surface area which is disposed in contact with the corresponding oval window on applying the prosthesis to the middle ear due to the continuously arched member (1).

## Claims

1. Prosthesis for complete ossicular replacement which, being envisaged to replace all the damaged ossicles of the middle ear, for the purpose of mitigating hypoacusia or hearing loss to a certain extent, having a member (1) which extends at one of its ends, considered proximal, into a fork (2) for anchorage thereof and, at the opposite end, into a base (3) having progressively increasing width, **characterised in that** the member (1) is a continuously arched member (1) for anchorage thereof to the corresponding medial zone of the hammer handle (7) underneath it without lifting the tympanic membrane, while the base (3) establishes a larger surface area which is disposed in contact with the corresponding oval window on applying the prosthesis to the middle ear due to the continuously arched member (1).

2. Total ossicular replacement prosthesis according to claim 1, **characterised in that** it consists preferably of titanium.

3. Total ossicular replacement prosthesis according to claim 1, **characterised in that** it consists of a biocompatible plastic material or materials comprised of fibres or nitinol.

## Patentansprüche

1. Prothese für einen vollständigen Gehörknöchelersatz, die dazu bestimmt ist, alle geschädigten Gehörknöchelchen des Mittelohrs ersetzen zu können, um Hypoakusie oder Hörverlust bis zu einem gewissen Grad zu mildern, mit einem Glied (1), das sich an einem seiner Enden, das als proximal betrachtet wird, in eine Gabel (2) zur Verankerung derselben und am anderen Ende in eine Basis (3) mit fortschreitend zunehmender Breite erstreckt, **dadurch gekennzeichnet, dass** das Glied (1) ein kontinuierlich gewölbtes Teil (1) zur Verankerung an der entsprechenden medialen Zone des Hammergriffs (7) umfasst, ohne die Trommelfellmembran anzuheben, während die Basis (3) eine größere Fläche bildet, die beim Anlegen der Prothese an das Mittelohr durch das kontinuierlich gewölbte Glied (1) in Kontakt mit dem entsprechenden ovalen Fenster angeordnet ist.

2. Prothese zum vollständigen Gehörknöchelersatz, nach Anspruch 1, **dadurch gekennzeichnet, dass** sie vorzugsweise aus Titan besteht.

3. Prothese für einen vollständigen Gehörknöchelersatz nach Anspruch 1, **dadurch gekennzeichnet, dass** sie aus biokompatiblem Kunststoff oder aus Fasern oder Nitinol bestehenden Materialien besteht.

## Revendications

1. Prothèse pour remplacement ossiculaire complet , étant envisagée pour remplacer tous les osselets endommagés de l'oreille moyenne, dans le but d'atténuer l'hypoacousie ou la perte auditive dans une certaine mesure, comprenant un membre (1) qui s'étend à une de ses extrémités, considérée proximale, en une fourche (2) d'ancrage, tandis que l'extrémité opposée s'étend en une base (3) ayant une largeur progressivement croissante, **caractérisée en ce que** le membre (1) est un membre arqué continu (1) pour son ancrage à zone médiane correspondante de la poignée de marteau (7), sous lequel et sans soulever la membrane du tympan, la base (3) établit une plus grande surface disposée en contact avec la fenêtre ovale correspondante lors de l'application de la prothèse à l'oreille moyenne dû au membre arqué continu (1).

2. Prothèse pour remplacement ossiculaire complet, selon la revendication 1, **caractérisée en ce qu'**elle est constituée de préférence de titane.

3. Prothèse pour remplacement ossiculaire complet, selon la revendication 1, **caractérisée en ce qu'**elle est constituée de matière plastique biocompatible ou de matériaux constitués de fibres ou de nitinol. Campo y antecedentes de la invención
